# EUROPÄISCHE PATENTSCHRIFT

(11) **EP 2 816 103 B1**
(45) Veröffentlichungstag und Bekanntmachung des Hinweises auf die Patenterteilung: **23.08.2017**
(21) Anmeldenummer: 14001927.4
(22) Anmeldetag: 03.06.2014
(51) Int. Cl.: C12M 1/22, B25J 9/00, C12M 1/00, C12M 1/26, G01N 35/00

(54) **VORRICHTUNG ZUM HANDHABEN VON PETRISCHALEN**
DEVICE FOR HANDLING PETRI DISHES
DISPOSITIF DE MANIPULATION DE BOÎTES DE PÉTRI

(30) Priorität: 04.06.2013 CH 10602013
(43) Veröffentlichungstag der Anmeldung: 24.12.2014
(73) Patentinhaber: LICONIC AG, 9493 Mauren (LI)
(72) Erfinder: Cosmas, Malin, 9463 Mauren (LI)
(74) Vertreter: Sutter, Kurt

(56) Entgegenhaltungen:
- EP-A1- 1 661 980
- EP-A2- 2 482 079
- DE-A1-102010 060 634

## Beschreibung

### Gebiet der Erfindung

Die Erfindung betrifft eine Vorrichtung zur Handhabung von Petrischalen, eine Lagervorrichtung mit einer derartigen Vorrichtung zur Handhabung von Petrischalen sowie ein verfahren zum Betrieb der Vorrichtung.

### Hintergrund

Petrischalen sind flache, runde, in der Regel durchsichtige Schalen mit einem Boden und einem den Boden übergreifendem Deckel, die in der Biologie, Medizin oder Chemie verbreitet zum Einsatz kommen. Dabei werden Petrischalen zur Kultivierung von Mikroorganismen und Zellkulturen genutzt.

Mikroorganismen werden lokal in ein Nährmedium im Boden eingebracht. Anschliessend werden die Petrischalen meist mit dem Deckel nach unten und dem Nährmedium nach oben inkubiert. Bei dieser Lagerung lastet das Gewischt der Platte auf dem Deckel, wodurch der Verschluss zwischen Deckel und Schale verbessert wird. Überschüssiges Wasser bildet sich nicht auf dem Nährboden, sondern sammelt sich im Deckel.

Während der Inkubationszeit wird das Wachstum der Kulturen mehrmals visuell inspiziert. In Anwendungen in denen mit grossen Zahlen von Platten gearbeitet wird, besteht Bedarf für eine Automatisierung dieses Vorgangs. Dabei wird nach bestimmten zeitlichen Vorgaben jeweils eine Petrischale dem Inkubator bzw. der Lagervorrichtung entnommen und einer Verarbeitungsvorrichtung, insbesondere einer finalysevorrichtung, zugeführt. Für die Inspektion sollen der Inspektionavorrichtung die Platten mit dem Nährboden nach unten zugeführt werden.

Zum Transferieren der Petrischalen zwischen Lager und Inspektionsvorrichtung wird eine Transfervorrichtung benötigt. Üblicherweise besitzt diese einen Schwenkarm, der an einer Wandung der Inkubationskammer bzw. Lagervorrichtung angeordnet ist. Eine Petrischale wird von einem Vakuumsauger, der am äusseren Ende des Schwenkarms angeordnet ist, erfasst und mittels einer Drehbewegung von 180 Grad aus dem Inkubator entnommen und gleichzeitig gewendet. Bei der Inspektionsvorrichtung muss der Vakuumsauger für die Inspektion entfernt werden.

Aus EP 2 482 079 ist eine Vorrichtung bekannt, in welcher lediglich der Boden der Petrischalen gewendet werden.

Andere Lösungen basieren darauf, dass die Petrischalen im Transportlift innerhalb des Inkubators gewendet werden. Hier besteht aufgrund der Gefahr des Plattenverlusts das Risiko einer Kontamination des ganzen Inkubators.

### Darstellung der Erfindung

Es stellt sich die Aufgabe, eine Vorrichtung zur Handhabung von Petrischalen, eine Lagervorrichtung mit einer derartigen Vorrichtung zur Handhabung von Petrischalen sowie ein Verfahren zum Betrieb der Vorrichtung bereitzustellen, welche hohen Durchsatz haben.

Diese Aufgabe wird von den Gegenständen unabhängigen Ansprüchen gelöst. Demgemäss besitzt die Vorrichtung einen um eine, insbesondere horizontale, Drehachse drehbaren Drehhalter. Am Drehhalter sind mindestens eine erste und eine zweite Halterung für Petrischalen angeordnet, wobei durch Drehen des Drehhalters die erste Halterung von einer unteren Position in eine obere Position und die zweite Halterung von der oberen Position in die untere Position gebracht und dadurch die in den Halterungen gehaltenen Petrischalen gewendet werden können. Diese Ausgestaltung, bei welcher mindestens zwei Petrischalen gleichzeitig gewendet und zwischen zwei Positionen hin- und herbewegt werden, erlaubt eine Verarbeitung mit hohem Durchsatz.

Die erste und die zweite Halterung sind vorzugsweise um 180° um die Drehachse herum angeordnet.

Vorteilhaft besitzt jede Halterung einen Deckelhalter zur Aufnahme des Deckels einer Petrischale, sowie ein Andruckelement, wobei das Andruckelement und der Deckelhalter radial gegeneinander bewegt werden können. Der Begriff "radial" ist in der Beschreibung und den Ansprüchen bezüglich der Drehachse des Drehhalters zu verstehen, d.h. er beschreibt ein Achse oder Richtung, welche senkrecht zur Drehachse steht und die Drehachse schneidet.

In einer besonders vorteilhaften Ausführung wird die radiale Bewegung von einem Spreizantrieb erzeugt, der beiden Halterungen gemeinsam ist und mit welchem die Andruckelemente radial nach aussen und so gegen die Deckel der in den Halterungen gehaltenen Petrischalen bewegt werden können.

Weiter weist jeder Deckelhalter vorteilhaft eine Deckel-Haltervorrichtung auf, mit welcher der Deckel ohne den Boden in der oberen Position festgehalten werden kann. Die Deckel-Haltervorrichtung kann Klemmorgane aufweisen, zwischen welchen der Deckel mittels Lateralkräften festgeklemmt werden kann. Unter "Lateralkräften" sind dabei Kräfte zu verstehen, die seitlich auf den Deckel wirken, d.h. von aussen gegen dessen Seitenwände gerichtet sind.

Die Vorrichtung kann einen Greifer aufweisen, mit welchem der Boden einer sich in der oberen Position befindlichen Petrischale ergriffen und aus dieser Position herausgenommen oder in diese Position eingeführt werden kann. Dieser Greifer kann vorteilhaft in einer Richtung parallel zur Drehrichtung des Drehhalters translatorisch bewegt werden und Klemmorgane aufweisen, zwischen denen der Boden durch Lateralkräfte festgeklemmt werden kann. Unter "Lateralkräften" sind dabei wiederum Kräfte zu verstehen, die seitlich auf den Boden wirken, d.h. von aussen gegen dessen Seitenwände.

Die Erfindung betrifft auch eine Lagervorrichtung zur Lagerung und Manipulation, z.B. Inspektion, von Petrischalen. Die Lagervorrichtung besitzt die oben erwähnte Vorrichtung zur Handhabung von Petrischalen und weiter ein Lager zur Aufnahme mehrerer Petrischalen mit dem Deckel nach unten und eine Verarbeitungsvorrichtung. Die Anlage ist so ausgestaltet, dass die Petrischalen mit der erwähnten Vorrichtung zur Handhabung von Petrischalen auf ihrem Transportweg zwischen dem Lager und der Verarbeitungsvorrichtung gewendet werden können.

Schliesslich betrifft die Erfindung auch ein Verfahren zum Betrieb der genannten Vorrichtung, welches die folgenden Schritte umfasst:
a) Die erste Halterung wird in die untere und die zweite Halterung in die obere Position gebracht;
b) eine erste Petrischale wird in der ersten Halterung und eine zweite Petrischale wird in der zweiten Halterung positioniert - dies kann vor, während oder nach Schritt a) stattfinden;
c) durch Drehen des Drehhalters um die Drehachse wird die erste Halterung mit der ersten Petrischale in die obere Position und die zweite Halterung mit der zweiten Petrischale in die untere Position gebracht, während der Deckel und der Boden jeder Petrischale gegeneinander gedrückt werden.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine schematische Draufsicht auf eine Lagervorrichtung für Petrischalen,
Fig. 2 eine Vorrichtung zur Handhabung von Petrischalen mit Drehhalter und Greifer,
Fig. 3 die Vorrichtung nach Fig. 2 in Schnittansicht,
Fig. 4 eine Ausführung der Andruckelemente mit Spreizantrieb,
Fig. 5 die Anordnung von Fig. 4 von der Seite her gesehen,
Fig. 6 eine Ausführung des Deckelhalters und
Fig. 7 eine Ausführung eines Greifers.

### Wege zur Ausführung der Erfindung

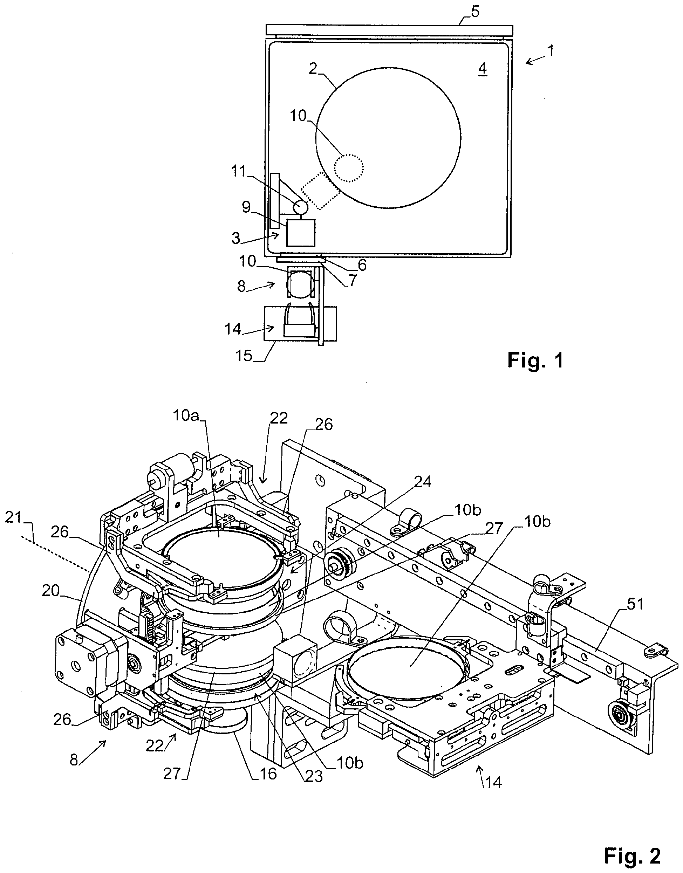

Fig. 1 zeigt eine Lagervorrichtung für Petrischalen. Diese umfasst einen Inkubator 1, in welchem ein Lager 2 in Form eines Karussells und eine Transporteinrichtung 3 angeordnet sind. Komponenten dieser Art sind z.B. in EP 2 482 079 beschrieben. Im Lager 2 sind die Petrischalen 10, welche jeweils einen Deckel und einen Boden besitzen, mit dem Deckel nach unten gelagert, während die zu inkubierende Kultur am oben liegenden Boden angeordnet ist.

Beim Inkubator 1 handelt es sich um einen Klimaschrank, in dessen Innenraum 4 eine vorgegebene Temperatur, Atmosphäre und/oder Luftfeuchte aufrecht erhalten werden kann. Auf einer Seite des Inkubators 1 ist eine Benutzertüre 5 angeordnet, über welche der Benutzer auf den Innenraum 4 zugreifen kann. Auf der der Benutzertüre 5 gegenüber liegenden Seite des Inkubators 1 befindet sich eine Schleusenöffnung 6, die mit einer motorisch betriebenen Schleusentüre 7 verschlossen werden kann.

Die Transporteinrichtung 3 dient dazu, die Petrischalen 10 durch die Schleusenöffnung 6 zwischen dem Lager 2 und einem ausserhalb des Inkubators 1 angeordneten Drehhalter 8 zu bewegen. Der Drehhalter 8 wird weiter unten im Detail beschrieben. Die Transporteinrichtung 3 besitzt eine Schaufel 9, welche jeweils eine Petrischale 10 aufnehmen kann. Die Schaufel 9 ist höhenverstellbar, um eine Achse 11 schwenkbar und radial zur Achse 11 ausfahrbar, Der Drehalter 8 ist derart ausserhalb der Schleusenöffnung 6 angeordnet, dass eine Petrischale 10 mit der Transporteinrichtung mittels linearer Translationsbewegung durch die Schleusenöffnung 6 in eine (weiter unten beschriebene) untere Position des Drehhalters eingeführt oder aus dieser entnommen werden kann, und zwar vorteilhaft über die die lineare Translationsbewegung, die durch horizontales Aus- und Einfahren der Schaufel 9 erzeugt wird. Die Translationsbewegung zum Beschicken des Drehhalters 8 verläuft parallel zu einer (ebenfalls weiter unten beschriebenen) Drehachse des Drehhalters.

Die ausserhalb des Inkubators 1 angeordneten Komponenten der Anlage werden in diesem Text und den Ansprüchen als "Vorrichtung zur Handhabung von Petrischalen" bezeichnet. Sie umfassen insbesondere den bereits erwähnten Drehhalter 8, einen Greifer 14, eine Analysevorrichtung 15 (oder, allgemeiner formuliert, eine Verarbeitungsvorrichtung) sowie Inspektionskamera 16 (Fig. 2). Diese Komponenten, welche im Folgenden genauer beschrieben werden, besitzen die folgenden Aufgaben:
- Der Drehhalter 8 dient dazu, die vom Lager 2 kommenden (oder dorthin zurückkehrenden) Petrischalen zu wenden, so dass ihr Boden nach unten zu liegen kommt und den Boden und den Deckel zu trennen, so dass der Boden vom Greifer 14 ergriffen werden kann.
- Der Greifer 14 dient dazu, den Boden einer Petrischale zur Analysevorrichtung 15 zu bringen, wo die Kultur z.B, optisch analysiert wird,
- Die Inspektionskamera 16 dient dazu, die Petrischale vor dem Trennen von Deckel und Boden auf Kontaminationen mit gefährlichen Keimen zu prüfen, so dass im Falle einer Kontamination das Öffnen der Petrischale unterdrückt und so ein Austreten unerwünschter Keime vermieden werden kann.
- Die Analysevorrichtung 15 inspiziert den Boden der Petrischale in geöffnetem Zustand.

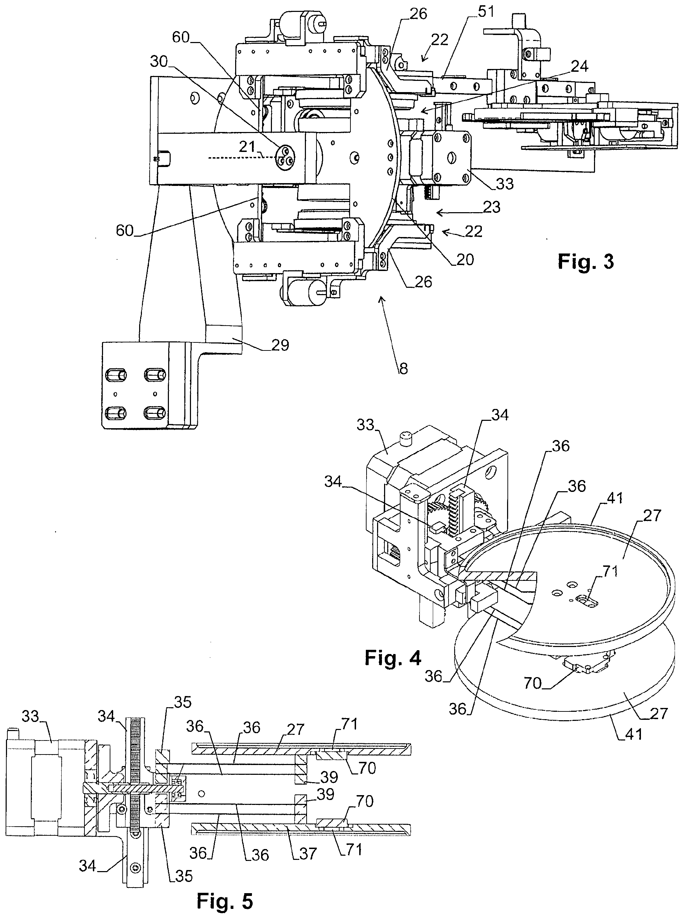

Ein möglicher Aufbau des Drehhalters 8 erschliesst sich aus den Figuren 2 bis 6. Er besitzt einen Drehkörper 20, der mittels einen Antriebs um eine horizontal verlaufende Drehachse 21 um mindestens 180° rotiert werden kann. Am Drehkörper 20 sind zwei Halterungen 22 angeordnet (Fig. 2, 3), welche jeweils eine Petrischale aufnehmen können. Die Halterungen 22 können durch Drehung des Drehkörpers 20 um die Drehachse 21 gedreht werden, und zwar so, dass jeweils eine davon sich in einer unteren Position 23 und die andere in einer oberen Position 24 befindet.

Wie aus Fig. 3 ersichtlich, ist ein Halter 29 vorgesehen, welcher an der Aussenwand des Inkubators 1 befestigt werden kann, und der den Drehhalter 8 in einem Lager 30 drehbar um die Drehachse 21 trägt.

Jede Halterung 22 besitzt einen Deckelhalter 26 zur Aufnahme und zum Festhalten des Deckels 10a einer Petrischale sowie ein Andruckelement 27, mit welchem der Deckel 10b gegen den Boden 10a gedrückt werden kann. Zu diesem Zweck können der Deckelhalter 26 und das Andruckelement 27 radial gegeneinander bewegt werden. In der dargestellten Ausführung sind hierzu die beiden Andruckelemente 27 radial beweglich, während die Deckelhalter 26 sich auf einer festen radialen Position befinden.

Um die Andruckelemente 27 zu bewegen, ist ein gemeinsamer Spreizantrieb vorgesehen. Dieser umfasst einen Spreizmotor 33, der am Drehkörper 20 angeordnet ist. Das Antriebsritzel des Spreizmotors 33 treibt zwei Zahnstangen 34 an (Fig. 4, 5), welche in einer Richtung senkrecht zur Drehachse 21 gegenläufig beweglich sind. An jeder Zahnstange 34 ist ein erster Federhalter 35 angeordnet, und in jedem Federhalter 35 sind zwei parallele Blattfedern 36 gehalten. Diese Blattfedern, welche über die ersten Federhalter 35 und die Zahnstangen 34 mit dem Spreizantrieb verbunden sind, erstrecken sich senkrecht zur Drehachse 21. Sie sind so angeordnet, dass ihre Oberflächennormalen (d.h. die oberflächennormalen auf die jeweils grössten zwei Oberflächen jeder Blattfeder) senkrecht zur Drehachse 21 stehen. Die dem ersten Federhalter 35 gegenüber liegenden Enden der Blattfedern 36 sind jeweils an einem zweiten Federhalter 39 befestigt, der seinerseits jeweils eines der Andruckelemente 27 trägt. Die einem Andruckelement 27 zugeordneten zwei Blattfedern 36 besitzen jeweils unterschiedliche Abstände zur Drehachse 21. Auf diese Weise entsteht eine elastische Lagerung für jedes Andruckelement, welche eine radiale Auslenkung der Andruckelemente erlaubt, aber ein Verkippen der Andruckelemente verhindert.

Jedes Andruckelement 27 wird von einer Platte gebildet, die einen hochgezogenen, kreisförmigen Rand 41 besitzt, welcher den Boden 10b der Petrischale in der oberen Position 24 seitlich führt.

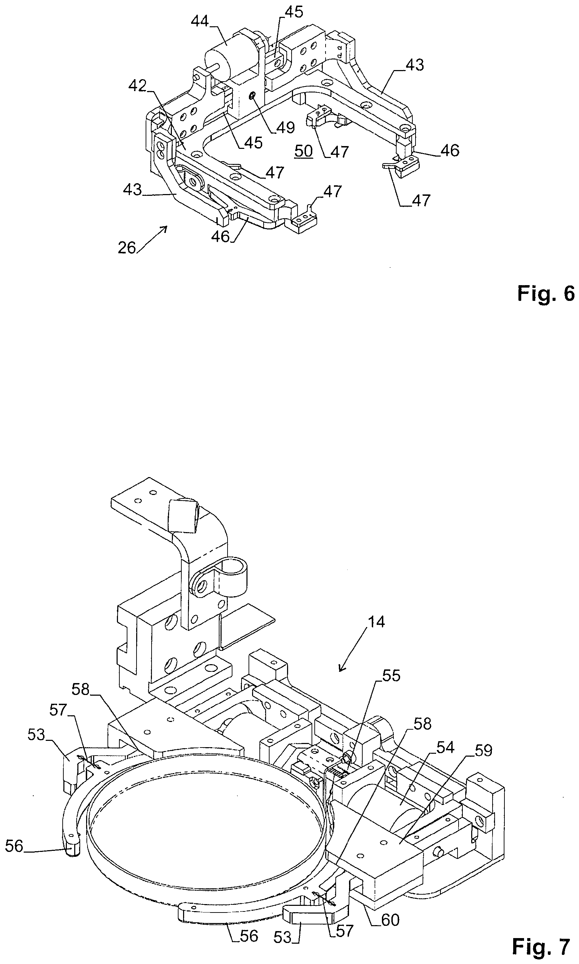

Wie insbesondere aus Fig. 6 ersichtlich, besitzt jeder Deckelhalter 26 einen Rahmen 42, der fest mit dem Drehhalter 8 verbunden ist. Am Rahmen 42 sind zwei gegeneinander bewegbare Arme 43 verschiebbar befestigt, die von einem Antrieb 44 synchron gegeneinander bewegt werden können. Um eine Synchronisierung der Bewegungen der Arme 43 sicherzustellen, sind an den Armen 43 Zahnstangen 45 angeordnet, welche an gegenüber liegenden Seiten eines Ritzels (siehe Ritzellager 49) eingreifen. Der Antrieb 44 ist als Linearantrieb ausgestaltet, mit welchem die Arme 43 gegen eine Federkraft auseinander gedrückt werden können.

An jedem Arm 43 ist federnd ein Finger 46 angeordnet, der zwei Gegenlager 47 trägt, welche so positioniert sind, dass sie den Deckel 10a einer in der Halterung angeordneten Petrischale radial von aussen stützen.

Um den Deckel einer Petrischale festzuhalten, werden die Arme 43 aufeinander zu bewegt, so dass die Spitzen der Finger 46 gegen die Aussenseite des Deckels drücken und diesen festklemmen.

Die Komponenten das in Fig. 6 dargestellten Deckelhalters 26 sind um eine Aussparung 50 herum angeordnet, durch welche mindestens 80%, insbesondere mindestens 90%, des Deckels der Petrischale radial von aussen observierbar sind. Auf diese Weise ist eine optische Inspektion durch die bereits erwähnte Inspektionskamera 16 möglich, wenn sich eine Petrischale in der unteren Position 23 befindet. Die Inspektionskamera 16 nimmt das Inkubat radial von aussen durch den Deckel 10a hindurch auf, um dieses zu inspizieren und um z.B. ein unerwartetes, durch Fremdkeime erzeugtes Wachstum, zu detektieren.

Um den Kontrast für die Inspektionskamera 16 zu verbessern, ist die der jeweiligen Petrischale zugewandte (petrischalenseitige) Seite jedes der plattenförmigen Andruckelemente 27 optisch homogen, d.h. die Reflektivität ändert sich über die Oberfläche um höchstens 10%. Insbesondere ist die petrischalenseitige Oberfläche dunkel, d.h. sie besitzt eine optische Reflektivität von weniger als 20%, insbesondere von weniger als 10%, bei mindestens einer Wellenlänge zwischen 300 und 1000 nm. Die genannte Wellenlänge ist diejenige Wellenlänge (bzw. derjenige Wellenlängenbereich), bei welchem die Inspektionskamera 16 ihre Messung durchführt.

Wie aus Fig. 2 und 3 ersichtlich, ist am Halter 29 eine Linearführung 51 angeordnet, an welcher der Greifer 14 in einer Richtung parallel zur Drehachse 21 verfahren werden kann.

Der Aufbau des Greifers 14 ergibt sich aus Fig. 7. Der Greifer besitzt Klemmorgane, um den Boden 10b der Petrischale in der oberen Position 24 durch Lateralkräfte festzuklemmen. Ähnlich wie beim Deckelhalter 26 sind die Klemmorgane an zwei Armen 53 gehalten, welche von einem Antrieb 54 synchron gegeneinander bewegt werden können. Um die Bewegung der Arme 53 zu synchronisieren, sind diese an Zahnstangen 55 befestigt, welche in die einander gegenüber liegenden Seiten eines Ritzels eingreifen.

Die Klemmorgane des Greifers 14 weisen Finger 56 auf, welche Über Blattfedern 57 federnd gehalten sind. Jeder Finger greift mit seinem dem Drehhalter 8 entgegen gesetzten Ende in eine Führung 58 ein. Diese Führung wird vom Spalt zwischen zwei beabstandeten Platten 59, 60 gebildet und beschränkt die Bewegung der Enden und somit der Finger 56 nach oben und unten, um so Beschädigungen zu vermeiden.

Der Greifer 14 ist so angeordnet, dass er den Boden 10b einer Petrischale in der oberen Position 24 ergreifen bzw, in die obere Position 24 ablegen kann.

Auf der dem Greifer 14 gegenüber liegenden Seite besitzt der Drehhalter 8 Ausnehmungen 60 (Fig. 3), durch welche die Transporteinrichtung 3 der Anlage jeweils auf die Petrischale in der unteren Position 22 zugreifen kann.

### Der Betrieb der beschriebenen Anlage ist wie folgt:

Muss eine Petrischale 10 dem Lager 2 entnommen und zur Analysevorrichtung 15 gebracht werden, wird das Lager 2 so gedreht, dass die Transporteinrichtung 3 auf die gewünschte Petrischale 10 zugreifen kann. Sie entnimmt mit ihrer Schaufel 9 die Petrischale 10 dem Lager 2. Die Schleusentüre 7 wird geöffnet und die Schaufel 9 wird in einer linearen Translationsbewegung durch die Schleusenöffnung 6 gefahren. Sie legt die Petrischale im Deckelhalter 26 der Halterung 22 in der unteren Position 23 ab. Der Deckel 10a wird von der Klemmvorrichtung des Deckelhalters 26 seitlich fixiert. Die Andruckelemente 27 werden gespreizt, so dass das untere Andruckelement gegen den Boden 10b der in der unteren Position 23 gelagerten Petrischale drückt und so den Boden 10b auf dem Deckel 10a fixiert.

Gleichzeitig, vorher oder nachher kann mit der Inspektionskamera 16 die Petrischale auf Fremdkeime hin inspiziert werden. Wir ein Fremdkeim erkannt, so wird z.B. Alarm gegeben und die Petrischale kann ausgeschieden werden.

Nun wird der Drehhalter 8 um die Drehachse 21 um 180° gedreht. Dabei wird die Petrischale gewendet, so dass sich nach der Drehung des Drehhalters 8 in der oberen Position 24 der Deckel 10a oben und der Boden 10b unten befindet. Gleichzeitig wird eine zweite Petrischale, welche sich allenfalls zuvor in der oberen Position befunden hat, nach unten bewegt und zurückgewendet.

Jetzt wird der Spreizantrieb 33 in Betrieb gesetzt, und die Andruckelemente 27 werden von ihrer äusserten Stellung in eine Zwischenstellung gebracht. Dadurch wird der Boden 10b der Petrischale in der oberen Position 24 abgesenkt und löst sich vom Deckel. Gleichzeitig wird die allfällige zweite Petrischale in der unteren Position 23 freigegeben, so dass sie z.B. von der Transporteinrichtung 3 erfasst und ins Lager 2 transferiert werden kann.

Nun fährt der Greifer 14 mit seinen Fingern 56 in die obere Position 24 ein und ergreift den Boden 10b der dortigen Petrischale. Danach wird der Spreizantrieb 33 nochmals betätigt, um die Andruckelemente 27 aus ihrer Zwischenstellung in eine radial innerste Stellung zu bringen. Dadurch wird der Rand 41 des oberen Andruckelements 27 unter den Boden 10b abgesenkt, so dass der Greifer 14 den Boden 10b aus der oberen Position ausfahren und zur Analysevorrichtung 15 bewegen kann, ohne mit dem Rand 41 zu kollidieren.

Der Greifer 14 bringt den Boden 10b sodann über die Analysevorrichtung 15, wo das Inkubat analysiert werden kann.

Um den Boden 10b bzw. die Petrischale wieder zurück ins Lager 2 zu bringen, wird der Boden 10b vom Greifer 14 zunächst wieder in die obere Position 24 zurückgebracht, die Andruckelemente 27 werden in die radial äusserste Stellung gespreizt, der Drehhalter 8 wird rotiert und die Transporteinrichtung 3 entnimmt die Petrischale aus der unteren Position 22 zwecks Rückführung ins Lager 2.

### Bemerkungen:

In der beschriebenen Ausführung kommen bei den Linearführungen mit Vorteil Kugelkettenlager zum Einsatz.

Zur Detektion der Anwesenheit von Petrischalen in den Halterungen 22 und/oder im Greifer 14 können geeignete Sensoren, wie z.B. optische Sensoren, eingesetzt werden. Ein Beispiel eines solchen Sensors 70 für die Halterungen 22 ist in Fig. 4 und 5 dargestellt. Er ist am Andruckelement 27 montiert und dazu ausgelegt, durch eine öffnung 71 eine optische Reflektionsmessung am Boden 10a der Petrischale durchzuführen.

In den obigen Ausführungen ist die anspruchsgemässe Vorrichtung zur Handhabung von Petrischalen ausserhalb des Inkubators 1 angeordnet. Grundsätzlich kann sich jedoch auch innerhalb des Inkubators 1 angeordnet sein.

Die Andruckelemente 27 sind frei positionierbar und ihre verschiedenen Stellungen können den jeweiligen Anforderungen angepasst werden. Insbesondere kann die radial äusserste Position an die Höhe der Beladung, d.h. an die Höhe der Petrischale, angepasst werden. Weiter können die Positionen der Andruckelemente an die Schwenkhöhe und an die Einfahrhöhe des Greifers angepasst werden.

Der Drehhalter 8 besitzt in den obigen Ausführungen zwei Halterungen 22 für Petrischalen. Denkbar sind auch Ausführungen mit mehr als zwei Halterungen, z.B. drei oder vier Halterungen, welche über den Umfang des Drehhalters 8 gleichmässig verteilt sind. In diesem Falle dreht sich der Drehhalter 8 pro Arbeitsschritt jeweils um weniger als 180°. Die Andruckelemente 27 sind in diesem Falle so ausgestaltet, dass sie nur jeweils die Petrischalen in der obersten und der untersten Position freigeben.

Wie erwähnt, sind die Arme 43, 53 vorteilhaft synchron gegeneinander bewegbar, was in der oben beschriebenen Ausführungen über Zahnstangen realisiert wird, die an gegenüber liegenden Seiten eines Ritzels eingreifen. Alternativ können auch andere mechanische (oder elektrische) Mittel zur Synchronisierung der Armpositionen eingesetzt werden, wie z.B. Kurvenscheiben oder Gelenke.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Vorrichtung zur Handhabung von Petrischalen, welche einen Boden und einen Deckel aufweisen, wobei die Vorrichtung dazu ausgestaltet ist, eine Petrischale mit unten liegendem Deckel zu empfangen, zumindest den Boden der Petrischale zu wenden und abzugeben oder umgekehrt, **dadurch gekennzeichnet, dass** die Vorrichtung einen um eine Drehachse (21) drehbaren Drehhalter (8) aufweist, an welchem mindestens eine erste und eine zweite Halterung (22) für Petrischalen angeordnet sind, wobei durch Drehen des Drehhalters (8) die erste Halterung (22) von einer unteren Position (23) in eine obere Position (24) und die zweite Halterung (22) von der oberen Position (24) in die untere Position (23) bringbar und die in den Halterungen (22) gehaltenen Petrischalen wendbar sind.

2. Vorrichtung nach Anspruch 1, wobei jede Halterung (22) einen Deckelhalter (26) zur Aufnahme des Deckels einer Petrischale aufweist, sowie ein Andruckelement (27), wobei das Andruckelement (27) und der Deckelhalter (26) bezüglich der Drehachse (21) radial gegeneinander bewegbar sind, um den Deckel der Petrischale gegen den Boden zu drücken.

3. Vorrichtung nach Anspruch 2, wobei die Andruckelemente (27) beider Halterungen (22) von einem gemeinsamen Spreizantrieb (33) radial nach aussen bewegbar sind.

4. Vorrichtung nach Anspruch 3, wobei jedes Andruckelement (27) über mindestens zwei parallele Blattfedern (36) mit dem Spreizantrieb (33) gekoppelt ist, wobei die Oberflächennormalen der Blattfedern (36) senkrecht zur Drehachse (21) stehen und die Blattfedern (36) unterschiedliche Abstände zur Drehachse (21) besitzen, derart, dass die Blattfedern eine elastische radiale Auslenkung ohne Kippung der Andruckelemente (27) erlauben.

5. Vorrichtung nach einem der Ansprüche 2 bis 4, wobei die Andruckelemente (27) petrischalenseitig von optisch homogenen Platten gebildet werden, insbesondere Platten mit einer optischen Reflektivität von weniger als 20% bei mindestens einer Wellenlänge zwischen 300 und 1000 nm.

6. Vorrichtung nach einem der Ansprüche 2 bis 5, wobei der Deckelhalter (26) eine Aussparung (50) aufweist, durch welche mindestens 80%, insbesondere mindestens 90%, des Deckels der Petrischale radial von aussen observierbar sind.

7. Vorrichtung nach Anspruch 6, wobei der Deckelhalter (26) Gegenlager (47) aufweist, wobei die Gegenlager (47) derart positioniert sind, dass sie den Deckel einer in der Halterung (22) angeordneten Petrischale radial von aussen stützen.

8. Vorrichtung nach einem der Ansprüche 2 bis 7, wobei jeder Deckelhalter (26) eine Deckel-Haltevorrichtung aufweist, mit welcher der Deckel ohne den Boden in der oberen Position (24) festhaltbar ist, und insbesondere wobei die Deckel-Haltervorrichtung Klemmorgane (46) aufweist, zwischen welchen der Deckel durch Lateralkräfte festklemmbar ist.

9. Vorrichtung nach einem der vorangehenden Ansprüche, welche weiter eine Inspektionskamera (16) aufweist, mit welcher eine in der unteren Position (23) befindliche Petrischale radial von aussen durch den Deckel inspizierbar ist.

10. Vorrichtung nach einem der vorangehenden Ansprüche, welche weiter einen Greifer (14) aufweist, mit welchem der Boden einer Petrischale aus der oberen Position (24) herausnehmbar oder in die obere Position (24) einführbar ist.

11. Vorrichtung nach Anspruch 10, wobei der Greifer (14) in einer Richtung parallel zur Drehachse (21) translatorisch bewegbar ist und Klemmorgane (56) aufweist, zwischen denen der Boden durch Lateralkräfte festklemmbar ist.

12. Vorrichtung nach einem der Ansprüche 8 oder 11, wobei die Klemmorgane an zwei Armen (43, 53) gehalten sind, wobei die Arme (43, 53) von einem Antrieb (44, 54) synchron gegeneinander bewegbar sind, und insbesondere wobei die beiden Arme (43, 53) an Zahnstangen (45, 55) angeordnet sind, welche an gegenüber liegenden Seiten eines Ritzels eingreifen.

13. Vorrichtung nach Anspruch 11, wobei die Klemmorgane Finger (56) zum Andrücken gegen den Boden aufweisen, wobei die Finger (56) federnd gehalten sind und an ihren dem Drehhalter (8) entgegen gesetzten Enden in eine Führung (58) eingreifen, wobei die Führung (58) die Bewegung dieser Enden nach oben und unten beschränkt.

14. Vorrichtung nach einem der Ansprüche 10 bis 13, wobei der Drehhalter (8) auf einer dem Greifer (14) gegenüber liegenden Seite Aussparungen (60) aufweist, durch welche Petrischalen in die untere Position (23) einführbar und/oder aus dieser entnehmbar sind.

15. Lagervorrichtung zur Lagerung und Manipulation von Petrischalen mit einer Vorrichtung zur Handhabung von Petrischalen nach einem der vorangehenden Ansprüche, umfassend ein Lager (2) zur Aufnahme mehrerer Petrischalen mit dem Deckel nach unten und eine Verarbeiturigsvorrichtung (15), wobei die Petrischalen mit der Vorrichtung zur Handhabung von Petrischalen auf einem Transportweg zwischen dem Lager (2) und der Verarbeitungsvorrichtung (15) wendbar sind.

16. Lagervorrichtung nach Anspruch 15, wobei das Lager (2) in einem Inkubator (1) angeordnet ist, welcher eine Schleusenöffnung (6) aufweist, und wobei im Inkubator (1) eine Transporteinrichtung (3) zum Bewegen von Petrischalen zwischen dem Lager (2) und dem Drehhalter (8) durch die Schleusenöffnung (6) angeordnet ist, wobei der Drehhalter (8) derart ausserhalb der Schleusenöffnung (6) angeordnet ist, dass mittels der Transporteinrichtung (3) eine Petrischale durch lineare Translationsbewegung durch die Schleusenöffnung (6) in die untere Position einführbar oder aus dieser entnehmbar ist, und insbesondere wobei die lineare Translationsbewegung parallel zur Drehachse (21) ist.

17. Verfahren zum Betrieb der Vorrichtung zur Handhabung von Petrischalen nach einem der Ansprüche 1 bis 14 umfassend die Schritte
a) die erste Halterung (22) wird in die untere und die zweite Halterung (22) in die obere Position gebracht,
b) eine erste Petrischale wird in der ersten Halterung (22) und eine zweite Petrischale wird in der zweiten Halterung (22) positioniert, und zwar vor, während oder nach Schritt a),
c) durch Drehen des Drehhalters (8) um die Drehachse (21) wird die erste Halterung (22) mit der ersten Petrischale in die obere Position und die zweite Halterung (22) mit der zweiten Petrischale in die untere Position gebracht, während der Deckel und der Boden jeder Petrischale gegeneinander gedrückt werden.

## Claims

1. Device for manipulating petri dishes having a base plate and a cover, wherein the device is adapted to receive a petri dish with the cover lying at the bottom, to turn around and to deliver, or vice versa, at least the base plate of the petri dish, **characterized in that** the device has a rotating holder (8) which is rotatable about a rotation axis (21), on which at least a first and a second support (22) for petri dishes are arranged, wherein the first support (22) is brought from a bottom position (23) into a top position (24) and the second support (22) is brought from the top position (24) into the bottom position (23) by rotating the rotating holder (8) and the petri dishes held in the supports (22) are reversible.

2. Device according to claim 1, wherein each support (22) has a cover holder (26) for receiving the cover of a petri dish, as well as a pressing element (27), wherein the pressing element (27) and the cover holder (26) are movable radially towards one another with respect to the rotation axis (21), in order to press the cover of the petri dish towards the bottom.

3. Device according to claim 2, wherein the pressing elements (27) of both supports (22) are movable radially outward by a common spread drive (33).

4. Device according to claim 3, wherein each pressing element (27) is coupled to the spread drive (33) via at least two parallel leaf springs (36), wherein the surface normals of the leaf springs (36) stand perpendicular to the rotation axis (21) and the leaf springs (36) have different distances to the rotation axis (21), in such a way that the leaf springs allow an elastic radial deflection without tilting the pressing elements (27).

5. Device according to one of the claims 2 to 4, wherein the pressing elements (27) are formed by optically homogenous plates on the side of the petri dishes, particularly plates with an optical reflectivity of less than 20% for at least a wavelength between 300 and 1000 nm.

6. Device according to one of the claims 2 to 5, wherein the cover holder (26) has a recess (50) through which at least 80%, particularly at least 90%, of the cover of the petri dish can be observed radially from outside.

7. Device according to claim 6, wherein the cover holder (26) has counter bearings (47), wherein the counter bearings (47) are positioned in such a way that they support the cover of a petri dish arranged in the support (22) radially from outside.

8. Device according to one of the claims 2 to 7, wherein each cover holder (26) has a cover holding device, by means of which the cover can be held in the top position (24) without the base plate, and particularly wherein the cover holding device has clamping organs (46), between which the cover is held by lateral forces.

9. Device according to one of the preceding claims, which furthermore has an inspection camera (16), by means of which a petri dish located in the bottom position (23) can be inspected radially from outside through the cover.

10. Device according to one of the preceding claims, which further has a gripper (14), by means of which the base plate of a petri dish can be taken out from the top position (24) or can be inserted into the top position (24).

11. Device according to claim 10, wherein the gripper (14) is movable in a translational way in a direction parallel to the rotation axis (21) and has clamping organs (56), between which the base plate can be clamped by lateral forces.

12. Device according to one of the claims 8 or 11, wherein the clamping organs are held by two arms (43, 53), wherein the arms (43, 53) are movable synchronously against one another by a drive (44, 54), and particularly wherein the two arms (43, 53) are arranged at toothed rods (45, 55) which engage at opposite sides of a pinion.

13. Device according to claim 11, wherein the clamping organs have fingers (56) for pressing towards the bottom, wherein the fingers (56) are held in a springy way and engage into a guide (58) with their ends which are opposite of the rotating holder (8), wherein the guide (58) limits the upward and downward movement of these ends.

14. Device according to one of the claims 10 to 13, wherein the rotating holder (8) has recesses (60) on a side which is opposite of the gripper (14), through which petri dishes are insertable into the bottom position (23) and/or removable therefrom.

15. Storage device for storing and manipulating petri dishes with a device for manipulating petri dishes according to one of the preceding claims, comprising a storage (2) for receiving multiple petri dishes with the cover arranged at the bottom and a processing device (15), wherein the petri dishes are reversible with the device for manipulating petri dishes on a transport path between the storage (2) and the processing device (15).

16. Storage device according to claim 15, wherein the storage (2) is arranged inside an incubator (1) which has a lock opening (6), and wherein a transport device (3) for moving petri dishes between the storage (2) and the rotating holder (8) through the lock opening (6) is arranged inside the incubator (1), wherein the rotating holder (8) is arranged outside the lock opening (6) in such a way that a petri dish is insertable by linear translatory movement through the lock opening (6) into the bottom position and is removable therefrom by means of the transport device, and particularly wherein the linear translation movement is parallel to the rotation axis (21).

17. Method for operating the device for manipulating petri dishes according to one of the claims 1 to 14, comprising the steps
a) the first support (22) is brought into the bottom position and the second support (22) into the top position,
b) a first petri dish is positioned in the first support (22) and a second petri dish is positioned in the second support (22), namely before, during or after step a),
c) the first support (22) with the first petri dish is brought into the top position and the second support (22) with the second petri dish is brought into the bottom position by turning the rotating holder (8), while the cover and the base plate of each petri dish are pressed against one another.

## Revendications

1. Dispositif de manipulation de boîtes de Petri ayant un fond et un couvercle, le dispositif étant adapté à recevoir une boîte de Petri avec le couvercle posé en bas, à tourner et délivrer, ou vice versa, au moins le fond de la boîte de Petri, **caractérisé en ce que** le dispositif a un porteur rotatif (8) qui est rotatif autour d'un axe de rotation (21), sur lequel au moins un premier et un deuxième support (22) pour des boîtes de Petri est arrangé, le premier support (22) étant apporté d'une position basse (23) dans une haute position (24) et le deuxième support (22) étant apporté de la haute position (24) dans la position basse (23) en tournant le porteur rotatif (8) et les boîtes de Petri tenues dans les supports (22) sont réversibles.

2. Dispositif selon la revendication 1, chaque support (22) ayant un support de couvercle (26) pour recevoir le couvercle d'une boîte de Petri, ainsi qu'un élément d'appui (27), l'élément d'appui (27) et le support de couvercle (26) pouvant être déplacés radialement l'un vers l'autre par rapport à l'axe de rotation (21), afin d'appuyer le couvercle de la boîte de Petri vers le fond.

3. Dispositif selon la revendication 2, les éléments d'appui (27) des deux supports (22) étant déplacés de manière radiale vers l'extérieur par un entrainement d'épandage (33).

4. Dispositif selon la revendication 3, chaque élément d'appui (27) étant couplé avec l'entrainement d'épandage (33) par au moins deux ressorts à lames (36) parallèles, les normales à la surface des ressorts à lames (36) étant orientées de manière perpendiculaire par rapport à l'axe de rotation (21) et les ressorts à lames (36) ayant des distances différentes à l'axe de rotation (21), de sorte que les ressorts à lames permettent une déviation élastique radiale sans incliner les éléments d'appui (27).

5. Dispositif selon l'une des revendications 2 à 4, les éléments d'appui (27) étant formés par des plaques optiquement homogènes du côté des boîtes de Petri, particulièrement des plaques avec une réflectivité optique inférieure à 20% pour au moins une longueur d'onde entre 300 et 1000 nm.

6. Dispositif selon l'une des revendications 2 à 5, le support de couvercle (26) ayant une cavité (50) à travers laquelle au moins 80%, particulièrement au moins 90%, du couvercle de la boîte de Petri peut être observé radialement de dehors.

7. Dispositif selon la revendication 6, le support de couvercle (26) ayant des contre-paliers (47), les contre-paliers (47) étant positionnés de sorte qu'ils supportent le couvercle d'une boite de Petri arrangée dans le support (22) radialement de dehors.

8. Dispositif selon l'une des revendications 2 à 7, chaque support de couvercle (26) ayant un dispositif de support de couvercle à l'aide duquel le couvercle peut être gardé dans la haute position (24) sans la plaque de base, et particulièrement le dispositif de support de couvercle ayant des organes de serrage (46), entre lesquels le couvercle est tenu par des forces latérales.

9. Dispositif selon l'une des revendications précédentes, ayant en outre une caméra d'inspection (16), à l'aide de laquelle une boîte de Petri située dans la position basse (23) peut être inspectée radialement de dehors à travers le couvercle.

10. Dispositif selon l'une des revendications précédentes, ayant en outre une pince (14), à l'aide de laquelle la plaque de base d'une boîte de Petri peut être enlevée de la haute position (24) ou peut être introduite dans la haute position (24).

11. Dispositif selon la revendication 10, la pince (14) étant mobile en translation dans une direction parallèle à l'axe de rotation (21) et ayant des organes de serrage (56), entre lesquels la plaque de base peut être serrée par des forces latérales.

12. Dispositif selon l'une des revendications 8 ou 11, les organes de serrage étant tenus par deux bras (43, 53), les bras (43, 53) étant mobiles de manière synchrone l'un contre l'autre par un entrainement (44, 54), et particulièrement les deux bras (43, 53) étant arrangés à des tiges crantées (45, 55) qui agissent des côtés opposés d'un pignon.

13. Dispositif selon la revendication 11, les organes de serrage ayant des doigts (56) pour appuyer vers le fond, les doigts (56) étant tenus de manière élastique et s'enclenchant dans un guidage (58) avec leurs extrémités qui sont opposées au porteur rotatif (8), le guidage (58) limitant le mouvement vers le haut et vers le bas de ces extrémités.

14. Dispositif selon l'une des revendications 10 à 13, le porteur rotatif (8) ayant des cavités (60) d'un côté qui est opposé à la pince (14), à travers lesquelles des boîtes de Petri sont insérées dans la position basse et/ou enlevées de la position basse.

15. Dispositif de stockage et de manipulation de boîtes de Petri avec un dispositif de manipulation des boîtes de Petri selon l'une des revendications précédentes, comprenant un stockage (2) pour recevoir des multiples boîtes de Petri avec le couvercle arrangé au fond et un dispositif de traitement (15), les boîtes de Petri étant réversibles avec le dispositif pour manipuler des boîtes sur un chemin de transport entre le stockage (2) et le dispositif de traitement (15).

16. Procédé selon la revendication 15, le stockage (2) étant arrangé à l'intérieur d'un incubateur (1) qui a une ouverture de verrouillage (6), et un dispositif de transport (3) pour déplacer de boîtes de Petri entre le stockage (2) et le porteur rotatif (8) à travers l'ouverture de verrouillage (6) étant arrangé à l'intérieur de l'incubateur (1), le porteur rotatif (8) étant arrangé à l'extérieur de l'ouverture de verrouillage (6) de sorte qu'une boîte de Petri peut être introduite par un mouvement linéaire en translation à travers l'ouverture de verrouillage (6) dans la position basse et peut être enlevée de la position basse à l'aide d'un dispositif de transport, et particulièrement le mouvement linéaire en translation étant parallèle à l'axe de rotation (21).

17. Procédé d'opération du dispositif pour manipuler de boîtes de Petri selon l'une des revendications 1 à 14, comprenant les étapes
a) le premier support (22) est apporté dans la position basse et le deuxième support (22) dans la haute position,
b) une première boîte de Petri est positionnée dans le premier support (22) et une deuxième boîte de Petri est positionnée dans le deuxième support (22), à savoir avant, pendant ou après l'étape a),
c) le premier support (22) avec la première boîte de Petri est apporté dans la haute position et le deuxième support (22) avec la deuxième boîte de Petri est apporté dans la position basse en tournant le porteur rotatif (8), et pendant cela le couvercle et la plaque de base de chaque boîte de Petri sont poussés l'une contre l'autre.
